(19) Europäisches Patentamt
European Patent Office
Office européen des brevets

(11) Publication number: **0 257 768 A1**

# (12) EUROPEAN PATENT APPLICATION

(21) Application number: 87306319.2

(22) Date of filing: 17.07.87

(51) Int. Cl.⁴: **C 07 C 121/66**, C 07 C 121/75, C 07 C 149/42, A 61 K 31/275

(30) Priority: 18.07.86 CS 5462/86

(43) Date of publication of application: 02.03.88 Bulletin 88/9

(84) Designated Contracting States: AT BE CH DE FR GB IT LI LU NL SE

(71) Applicant: SPOFA Spojené Podniky Pro Zdravotnickou Vyrobu, Husinecká 11 a, Prag 3 (CS)

(72) Inventor: Blaha, Ludvik, Na Neklance 1952, Praha (CS)
Inventor: Rajsner, Miroslav, Pod Karlovem 10, Praha (CS)
Inventor: Helfert, Ivan, Slana 785, Praha (CS)
Inventor: Trcka, Vaclav, Jungmannova 5, Praha (CS)

(74) Representative: Wotherspoon, Graham et al, FITZPATRICKS 4 West Regent Street, Glasgow G2 1RS Scotland (GB)

## (54) 2-Isopropyl-2-phenyl-5-(N-methyl-2-phenylethylamino)-valeronitrile derivatives and processes for their preparation.

(57) 2-Isopropyl-2-phenyl-5-(N-methyl-2-phenylethylamino)-valeronitrile derivatives of the general formula I

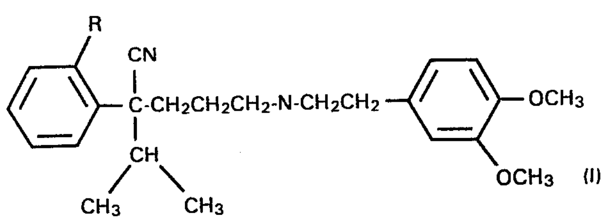

(I)

wherein R is a methyl, chlorine, bromine, $C_1$ to $C_4$ alkoxyl or methylthio group, preferably administered in the form of soluble acid addition salts, notably the 2-methylphenyl analog as its hydrochloride, possess significant calcium-antagonistic and cardiac antiarrythmic activity.

Preparation is by alkylation of the corresponding butyronitrile derivative (obtained from the respective substituted benzylcyanide by alkylation with isopropylbromide) by 3,3-diethoxypropylchloride, subsequent acidic hydrolysis of the 5,5-diethoxy-2-isopropyl-2-phenylvaleronitrile derivative obtained to the appropriate aldehyde, and reductive alkylation of this aldehyde with N-methylhomoveratrylamine by catalytic hydrogenation over a platinum or palladium catalyst or chemical reduction using formic acid. The resulting base is optionally neutralized with a pharmaceutically acceptable organic or inorganic acid, e.g. hydrochloric or fumaric acid, into the corresponding acid addition salt.

EP 0 257 768 A1

0257768

# 2-Isopropyl-2-phenyl-5-(N-methyl-2-phenylethylamino)valeronitrile derivatives and processes for their preparation

The invention relates to 2-isopropyl-2-phenyl-5-(N-methyl-2-phenylethylamino)valeronitrile derivatives of the general formula I

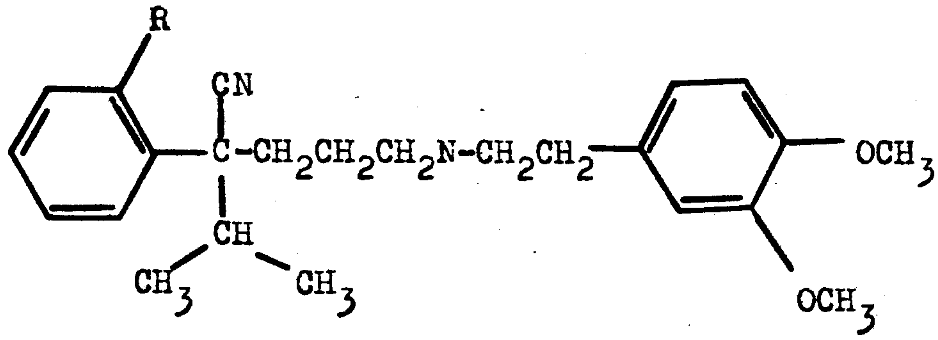

(I).

in which R is a methyl, chlorine, bromine, $C_1$ to $C_4$ alkoxyl or methylthio group, their addition salts with pharmaceutically acceptable inorganic or organic acids and to processes for their preparation.

The pertinent literature (Belg. pat. 615 861 (Chem. Abstr. 59, 13892 (1963)); Ger. pat. 1 158 083 (Chem. Abstr. 60, 5403 (1964)); Neth. Appl. 6 610 730 (Chem. Abstr. 67, 53899 (1967)); Brit. pat. 1 192 625, 1 202 499 and 1 202 500; Fr. pat. M 6911 (Chem. Abstr. 74, 125215 (1971)); Ger. Offen. 2 059 985 and 2 059 923, US pat. 3 957 845 (Chem. Abstr. 85, 177096 (1976)); Eur. pat. Appl. EP 64 158 (Chem. Abstr. 160 449, 98 (1983)); Ger. Offen. 3 121 766 (Chem. Abstr. 98, 107008 (1983)) describes numerous valeronitrile derivatives of related chemical structure. From among these known compounds 2-(3,4-dimethoxyphenyl)-2-isopropyl-5-(N-methyl-N-homoveratrylamino)valeronitrile and its 2-(3,4,5-trimethoxyphenyl) analog (respectively verapamil and gallopamil, nonproprietory generic names) are in clinical use for the treatment of coronary heart disease, certain cardiac arrhythmias and recently also hypertension.

Quite unexpectedly it was found that new valeronitrile derivatives of formula I, which are distinguished from the known structures by the fact that the substituent R on the benzene nucleus is attached in the position ortho with regard to the aliphatic connecting chain, are substantially less toxic than verapamil. Thus, their $LD_{50}$ values in mice on i.v. (intravenous) administration are in the range of from 20 to 40 mg/kg as compared to verapamil $LD_{50}$ of 5 mg/kg (animal and route same).

Biological properties of the subject compounds were evaluated both on isolated animal organs and in vivo, in experimental animals. Thus, for instance, the antiarrhythmic, negative inotropic (diminishing the heart contractility) and calcium ions blocking effects were tested on isolated electrically stimulated rabbit heart atria by determining respectively the prolongation of the functional refractory period, the decrease of heart contractility and the blockade of calcium-induced heart contractions dependently on rising concentration of the substance under test in the nutrient medium of the organ tissue culture. The calcium ions blocking effect was also evaluated by following the inhibition of calcium-induced contractions of isolated rabbit aortas. In vivo tests of the antiarrhythmic activity were performed in rats on experimental model arrhythmias induced by i.v. administration of aconitin, acetylcholine or calcium chloride. In almost all of these tests the subject valeronitrile derivatives elicited beneficial effects, predominantly comparable or even higher than those of verapamil used as a structurally related reference compound.

The most advantageous properties were found in the compound of formula I, in which R is a methyl; this compound was tested in the form of its hydrochloride (mepamil, nonproprietory preliminary generic name).

Mepamil elicits a markedly pronounced calcium ions blocking effect on isolated rabbit atria; also its calcium-antagonistic action on isolated smooth muscle of blood vessel wall is significant. The corresponding $ED_{50}$ values on rabbit atria and aortas are respectively $4 \cdot 10^{-3}$ and $9.3 \cdot 10^{-2}$ μmole/ml. On isolated rabbit atria mepamil elicits prolongation of the functional refractory period and has a favourably lower negative inotropic effect as compared to verapamil; the respective $ED_{50}$ values of the two agents are $1.2 \cdot 10^{-2}$ and $3.6 \cdot 10^{-3}$ μmole/ml. Mepamil has antiarrhythmic properties in experimentally induced arrhythmias both in vitro (e.g. on isolated rabbit atria) and in vivo, in experimental animals. Thus, it blocks barium chloride arrhythmia in rabbits, lowers the incidence of arrhythmias on i.v. administration of aconitin in rats and efficiently suppresses heart rhythm disorders in the same animals on rapid i.v. injection of acetylcholine; it also prevents fibrilation of heart ventriculi on i.v. administration of calcium chloride.

In haemodynamic studies in dogs and monkeys mepamil both on oral and i.v. administration lowers the systolic and especially the diastolic blood pressure, substantially reduces the heart rate and increases the blood flow rate in arteria carotis and particularly in coronary sinus; it increases the cardiac output and simultaneously diminishes the peripheral vascular resistance. Thus, in experiments in anaesthetized dogs mepamil doses ranging from 0.45 to 4.5 mg/kg i.v. reduce the systolic and diastolic blood pressure (respectively by up to 15 and 50%) as well as the heart rate (by up to 30%). Verapamil in similar doses elicits somewhat more pronounced effects, but when administered in doses of above 1 mg/kg i.v. it markedly lowers the myocardial contractility and induces atriaventricular block. In dogs a mepamil dose of 0.45 mg/kg i.v. substantially increases the blood flow rate in coronary sinus (by as much as 90% for a short time period). In conscious Macaca mulatta (Rhesus) monkeys an oral dose of 45 mg/kg has a mild hypotensive effect in animals with increased initial blood pressure.

Intravenously a dose of 4.5 mg/kg of the agent produces a short decrease both in systolic and diastolic blood pressure and lowers the heart rate in monkeys. Also, in DOCA-hypertensive rats a dose of 45 mg/kg p.o. has a significant hypotensive effect.

Changes in left ventricular pressure in dogs vs. time curve derivation confirm that mepamil has only a slight negative inotropic effect; this, on the contrary, is adversely high in verapamil.

The in vitro carcinogenity test of mepamil was negative, i.e. no signs of carcinogenic properties were observed. No mutagenic effect on Salmonella typhi murium was detectable.

In the short-time toxicity test in mice mepamil is five-times less toxic on i.v. administration and twice less toxic on oral ingestion as compared to verapamil. Thus, $LD_{50}$ of mepamil is 28 mg/kg intravenously and 620 mg/kg orally; for comparison, $LD_{50}$ of verapamil is 5 mg/kg i.v. During the four-week toxicity test in rats the administration of mepamil in daily doses of 25 and 100 mg/kg p.o. QD (on each day) elicited no significant differences in growth curves or adverse biochemical or histological changes in comparison with the control group animals.

The above summarized pharmacological assay results allow one to expect that the subject valeronitrile derivatives of formula I can find therapeutic use in similar medical indications as verapamil, i.e. especially in the treatment of coronary heart disease and certain arrhythmias; higher safety, namely substantially lower incidence of undesired side effects in the course of therapy, is expected in view of the reported slight toxicity and significantly diminished negative inotropic effect of the subject agents.

For the purpose of therapeutic use the active substances can be formulated into convenient medicinal dosage forms such as e.g. tablets, coated tablets, capsules, injections, sprays or transdermally acting plasters.

The subject valeronitrile derivatives of the general formula I can be prepared from the respective o-substituted benzylcyanides that predominantly, with the exception of 2-bromobenzylcyanide, 2-methylthiobenzylcyanide and 2-butoxybenzylcyanide, are known compounds; the route of preparation of the named new starting materials from the corresponding o-substituted benzylalcohols or benzylchlorides is described hereinbelow in the respective examples. The o-substituted benzylcyanides are alkylated with isopropylbromide in the presence of sodium amide in an inert organic solvent, e.g. ethyleneglycol dimethylether, to give butyronitrile derivatives of the general formula II

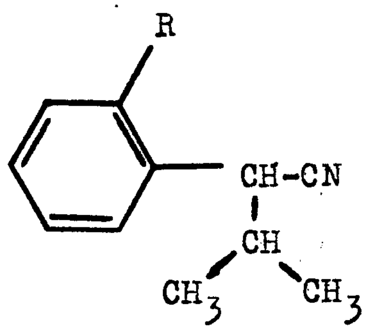

(II)

in which R is the same as in formula I. The alkylation is advantageously carried out by dropping a solution of the resulting o-substituted benzylcyanide in the above solvent to a toluenic sodium amide suspension diluted with ethyleneglycol dimethylether under cooling to a temperature below 10°C, subsequent stirring of the mixture at ambient temperature until the metalation is complete and dropping in a solution of isopropylbromide in ethyleneglycol dimethylether under cooling the reaction mixture to a temperature below 10°C. Finally, the alkylation is completed by stirring at room temperature for about 5 hours. The product is isolated conveniently by short agitation of the reaction mixture with an excess of water and subsequent extraction of the formed intermediary product into a suitable water-immiscible solvent, e.g. benzene or toluene,

and distilling off the solvent; the obtained material is purified by distillation under reduced pressure.

The butyronitrile derivatives of formula II are subsequently allowed to react with 3,3-diethoxypropylchloride in an inert organic solvent in the presence of an alkaline condensing agent under the formation of acetals of the general formula III

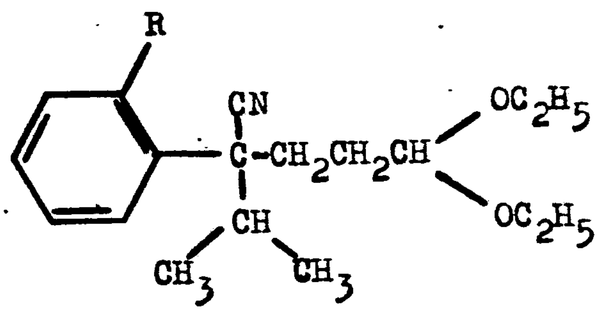

(III)

in which R is the same as in formula I. Suitable inert organic solvents are aromatic hydrocarbons, e.g. benzene, toluene or commercial xylene, tertiary amides, e.g. dimethylformamide, or higher-boiling aliphatic ethers, e.g. ethyleneglycol dimethylether. Preferable alkaline condensing agent is sodium hydride or sodium amide. The alkylation reaction is advantageously carried out by dropping a toluenic sodium amide suspension to a boiling solution of the preceding butyronitrile derivative of formula II and 3,3-diethoxypropylchloride in anhydrous toluene and refluxing the mixture for a period of 4 to 10 hours. The so-formed acetal of formula III is isolated upon cooling the reaction mixture by short agitation with water, separation of the toluenic phase and removal of the solvent by distillation; the obtained crude product is purified by distillation under reduced pressure.

In the subsequent reaction step the resulting acetals of formula III are subjected to mild acidically catalyzed hydrolysis to yield aldehydes of the general formula IV

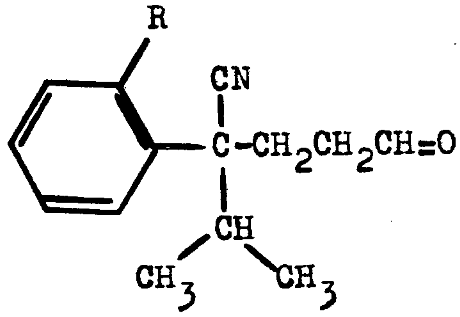

(IV)

in which R is the same as in formula I. This hydrolysis can be conveniently effected in aqueous acetone or wet ether in the presence of an inorganic, e.g. sulfuric or hydrochloric, or organic, e.g. p-toluenesulfonic, trifluoroacetic or oxalic acid, at a temperature of up to 50°C, and requires a period of approx. 2 to 10 hours. A preferable procedure consists in refluxing a stirred mixture of the acetal of formula III and oxalic acid dihydrate in acetone to a temperature of 30 to 40°C for a period of 3 to 5 hours. The liberated aldehyde of formula IV is isolated by neutralization of the oxalic acid with an aqueous potassium carbonate solution, separation of the potassium oxalate precipitate, removal of the solvent by distillation, short stirring of the residue with water and an appropriate water-immiscible solvent, e.g. ether, separation of phases and removal of the solvent.

The so obtained crude aldehydes of formula IV can be used immediately, without separate additional purification, for the final stage of the synthesis, reductive alkylation of the commercially available N-methylhomoveratrylamine under the formation of the desired valeronitrile derivatives of the general formula I. This reductive alkylation can be effected either by hydrogenation of a solution of these reactants in a $C_1$ to $C_4$ alkanol over platinum dioxide or palladium on a suitable carrier substrate, e.g. active carbon, at a temperature of 20 to 40°C or by chemical reduction with the use of formic acid as the reducing agent in an appropriate inert organic solvent, e.g. benzene,

toluene, a $C_1$ to $C_4$ alkanol, chloroform, ethyl acetate or aqueous dimethylformamide, at a temperature ranging from approx. 20°C to the boiling temperature of the reaction mixture. Advantageous procedure consists in refluxing a mixture of the aldehyde, N-methylhomoveratrylamine and 90% formic acid in toluene until the evolution of carbon dioxide is complete. The desired final product is isolated on cooling the reaction mixture by extraction into dilute sulfuric or hydrochloric acid, subsequent alkalization of the separated aqueous phase, e.g. with a sodium hydroxide solution, extraction of the so liberated oily base into a suitable water-immiscible organic solvent, e.g. benzene, toluene, ether, chloroform or dichloromethane, and removal of the solvent by evaporation. The so obtained bases of formula I can be converted by neutralization with appropriate inorganic or organic acids into the corresponding acid addition salts.

Starting materials required for the preparation are known substances available by methods described in the pertinent literature.

Further particulars of the procedure are illustrated by the following non-limitative examples.

Example 1

2-Bromobenzylcyanide

A mixture of phosphorus pentoxide (89.3 g) and o-bromophenylacetamide (67.4 g) is thoroughly homogenized and heated under reduced pressure of about 1.3 kPa to give crude 2-bromobenzylcyanide which distills off the mixture and is collected on cooling (yield 53.3 g). The product is dissolved in ether (200 ml), the solution is washed with water and an aqueous sodium hydrogencarbonate solution until neutral reaction, dried over anhydrous magnesium sulfate and the solvent is evaporated. The residue is distilled under reduced pressure to give pure 2-bromobenzylcyanide (49.5 g,

80.2% of theory), b.p. 132 to 133°C at 1.33 kPa, m.p. 20.5°C.

Example 2

2-Methylthiobenzylcyanide

To a solution of 2-methylthiobenzylalcohol (33.3 g) and pyridine (3.3 ml) in anhydrous benzene (230 ml) is added dropwise, under stirring and cooling below 15°C, 46.6 g of thionylchloride. The reaction mixture is boiled for 1 hour under reflux cooling and stirring, then it is cooled and poured into a stirred mixture of ice (50 g) and water (100 ml). After short, thorough agitation the benzene layer is separated, washed with cold water, an aqueous sodium hydrogencarbonate solution and water until neutral reaction, dried over anhydrous magnesium sulfate and the solvent is evaporated under reduced pressure to yield 38.1 g of crude 2-methylthiobenzylchloride. The product is diluted with ethanol (35 ml) and dropped under stirring, in the course of about 40 minutes, into an aqueous sodium cyanide (13.5 g) solution warmed on a boiling water bath. The mixture is refluxed for 4 hours, on cooling it is diluted with water (480 ml) and the separated oily substance is extracted into ether. The organic extract is washed with water until neutral reaction, dried over anhydrous magnesium sulfate and the solvent is evaporated. The residue is distilled under reduced pressure to give 2-methylthiobenzylcyanide (22.7 g, 64.4%), b.p. 88 to 89°C at 26.6 Pa.

Example 3

2-Butoxybenzylcyanide

To a solution of sodium cyanide (5.3 g) in water (5 ml) is dropped within about 40 minutes, under stirring and heating on a boiling water bath, a solution of 2-butoxybenzyl chloride (16.8 g) in ethanol (14 ml). The mixture is boiled under reflux for 4 hours. On cooling it is diluted with water (80 ml), the formed dark material is

extracted into ether, the extract is washed with water until neutral reaction, dried over anhydrous magnesium sulfate and the solvent is evaporated. The residue is distilled under reduced pressure to yield 2-butoxybenzylcyanide (7.4 g) 46.2%), b.p. 140 to 142°C at 0.78 kPa.

Example 4

2-(2-Chlorophenyl)-3-methylbutyronitrile

To a mixture of a 50% toluenic sodium amide suspension (17.9 g) and ethyleneglycol dimethylether (33 ml) is dropped during 20 minutes, under cooling to a temperature of 0 to 5°C, a solution of 2-chlorobenzylcyanide (28.9 g) in the same solvent (40 ml). The reaction mixture is stirred for 3 hours at about 20°C. On cooling with ice water a solution of isopropylbromide (30.6 g) in ethyleneglycol dimethylether (24 ml) is dropped in with stirring within 15 minutes; the mixture is maintained by external cooling at about 10°C. Stirring is continued for 30 minutes at about 15°C and thereafter for 5 hours at ambient temperature (20 to 23°C). The mixture is diluted with water (500 ml) and the product is extracted into benzene. The extract is washed with water, a 4% sodium hydroxide solution, water, 10% sulfuric acid and water until neutral reaction, dried over anhydrous magnesium sulfate, the solvent is evaporated under reduced pressure and the residue is distilled to give 33.6 g (91%) of the title compound, b.p. 130 to 132°C at 1.56 kPa.

Similar procedure yields

2-(2-bromophenyl)-3-methylbutyronitrile (89%),
b.p. 135°C/1.3 kPa,

2-(2-methylthiophenyl)-3-methylbutyronitrile (67.2%),
b.p. 98°C/26.6 Pa,

2-(2-methoxyphenyl)-3-methylbutyronitrile (90%),
b.p. 129 to 131°C/1.1 kPa,

2-(2-butoxyphenyl)-3-methylbutyronitrile (74.7%),
b.p. 115°C/0.12 kPa, and

2-(2-methylphenyl)-3-methylbutyronitrile (92%),

b.p. 112 to 114°C/0.53 kPa.

Example 5

5,5-Diethoxy-2-isopropyl-2-(2-methylphenyl)valeronitrile

To a boiling solution of 2-(2-methylphenyl)-3-methylbutyronitrile (207.9 g) and 86% 3,3-diethoxypropylchloride (154 g) in anhydrous toluene (1250 ml) is dropped during about 50 minutes under stirring 107.7 g of a 50% toluenic sodium amide suspension ($NaNH_2$ content 53.8 g) and the mixture is refluxed under continuous stirring for 6 hours. On subsequent cooling the stirred mixture is diluted with water (300 ml), the toluenic portion is separated and the aqueous phase is extracted with toluene. The organic solutions are combined, washed with water and saturated brine until neutral reaction, dried over anhydrous sodium sulfate, the solvent is evaporated under reduced pressure and the residue is distilled to give 302.9 g (83.2%) of 5,5-diethoxy-2-isopropyl-2-(2-methylphenyl)valeronitrile, b.p. 102 to 104°C at 4 Pa.

Similar procedure yields

5,5-diethoxy-2-isopropyl-2-(2-chlorophenyl)valeronitrile (76.3%), b.p. 101°C/2.66 Pa,

5,5-diethoxy-2-isopropyl-2-(2-bromophenyl)valeronitrile (67.9%), b.p. 130 to 131°C/26.6 Pa,

5,5-diethoxy-2-isopropyl-2-(2-methylthiophenyl)valeronitrile (71.7%), b.p. 122°C/4.0 Pa,

5,5-diethoxy-2-isopropyl-2-(2-methoxyphenyl)valeronitrile (60%), b.p. 116 to 117°C/1.3 Pa, and

5,5-diethoxy-2-isopropyl-2-(2-butoxyphenyl)valeronitrile (59%), b.p. 132°C/6.7 Pa.

Example 6

2-(2-Methylphenyl)-2-isopropyl-5-oxovaleronitrile

To a solution of 5,5-diethoxy-2-isopropyl-2-(2-methylphenyl)valeronitrile (267 g) in acetone (2000 ml) is added under stirring a solution of oxalic acid dihydrate

(121.7 g) in water (730 ml) and the mixture is warmed under continuous stirring for 4 hours at 40°C. Thereafter it is cooled and under external cooling to 5°C adjusted by the addition of a 50% aqueous potassium carbonate solution to pH within the range of 6 to 6.5. The formed potassium oxalate precipitate is filtered off and washed with acetone (500 ml). From the filtrate the solvent is evaporated under reduced pressure (bath temperature about 30°C). The residue is mixed with water (600 ml) and ether (500 ml), the separated aqueous phase is extracted with ether (2 x 300 ml), the combined organic solutions are washed with aqueous sodium hydrogencarbonate, water and saturated brine until neutral reaction, dried over anhydrous magnesium sulfate and the solvent is evaporated (bath temperature about 30°C) under reduced pressure, finally at about 1.3 kPa, to give 203 g of the oily crude product (title compound content 93.2% as determined by gas-liquid chromatography); the obtained material is used without separate purification for the subsequent reductive alkylation.

Similar procedure yields 2-(2-chlorophenyl)-2-isopropyl-5-oxovaleronitrile, 2-(2-bromophenyl)-2-isopropyl-5-oxovaleronitrile, 2-(2-methoxyphenyl)-2-isopropyl-5-oxovaleronitrile, 2-(2-butoxyphenyl)-2-isopropyl-5-oxovaleronitrile and 2-(2-methylthiophenyl)-2-isopropyl-5-oxovaleronitrile in the form of oily liquids; these are analyzed for the pure substance content by gas-liquid chromatography and used for further preparation.

Example 7

2-Isopropyl-2-(2-methylphenyl)-5-(N-methylhomoveratrylamino)-valeronitrile

Procedure A

A mixture of crude 2-isopropyl-2-(2-methylphenyl)-5-oxovaleronitrile (6.9 g, content 93.2%), N-methylhomoveratrylamine (5.9 g), platinum dioxide (0.1 g) and methanol (150 ml) is shaken in a hydrogenation vessel under hydrogen

until discontinuation of the gas absorption (for about 40 minutes). Platinum metal formed by reduction of the oxide is separated, washed with methanol and the solvent is evaporated from the filtrate under reduced pressure. The residue is mixed with water (120 ml) and ether (90 ml), the mixture is acidified with dilute hydrochloric acid, the separated aqueous phase is extracted with ether and alkalized with a 4% sodium hydroxide solution. The liberated oily base is extracted into ether, the extract is washed with water and saturated brine, dried over anhydrous sodium sulfate and the solvent is evaporated to give 11.2 g (91.4%) of the oily base. The obtained product is dissolved in a mixture of ether (30 ml) and 2-propanol (5 ml) and the solution is acidified with an etheric hydrogen chloride solution to yield crystalline hydrochloride, m.p. 159 to 161°C (from 2-propanol).

Similar procedure affords

2-(2-chlorophenyl)-2-isopropyl-5-(N-methylhomoveratrylamino)-valeronitrile (yield 77%); hydrochloride, m.p. 162 to 164°C (2-propanol),

2-isopropyl-2-(2-methoxyphenyl)-5-(N-methylhomoveratrylamino)valeronitrile (yield 85%); hydrochloride, m.p. 147 to 149°C (2-propanol), and

2-(2-butoxyphenyl)-2-isopropyl-5-(N-methylhomoveratrylamino)-valeronitrile (yield 80%), b.p. 195 to 197°C/1.33 Pa; hydrogenfumarate, m.p. 133 to 134°C (2-propanol - ether).

Procedure B

A mixture of 2-isopropyl-2-(2-methylphenyl)-5-oxovaleronitrile (201.8 g, content 93%), N-methylhomoveratrylamine (159.8 g), 90% formic acid (44.25 g) and toluene (1200 ml) is slowly warmed under stirring to the boiling temperature and gently boiled under reflux until discontinuation of the evolution of carbon dioxide (for about 1 hour). On cooling the reaction mixture the product is extracted into dilute hydrochloric acid and water, the

combined acidic aqueous solutions are washed with ether and alkalized with aqueous sodium hydroxide solution. The liberated oily base is extracted into ether, the extract is washed with water and saturated brine, dried over anhydrous sodium sulfate and ether is evaporated to give the oily product (293 g, 87.4 % of theory). Similarly as described in the preceding paragraph for procedure A the obtained base is converted into the hydrochloride (313 g, 85.7%) melting at 154 to 158°C; on recrystallization from 2-propanol pure hydrochloride has a m.p. of 159 to 161°C.

Similar procedure affords

2-(2-bromophenyl)-2-isopropyl-5-(N-methylhomoveratrylamino)-valeronitrile (yield 92%); hydrochloride, m.p. 155 to 156°C (2-propanol), and

2-isopropyl-2-(2-methylthiophenyl)-5-(N-methylhomoveratrylamino)valeronitrile (yield 80%), b.p. 201 to 203°C/2.7 Pa.

Claims

1. 2-Isopropyl-2-phenyl-5-(N-methyl-2-phenylethylamino)-valeronitrile derivatives of the general formula I

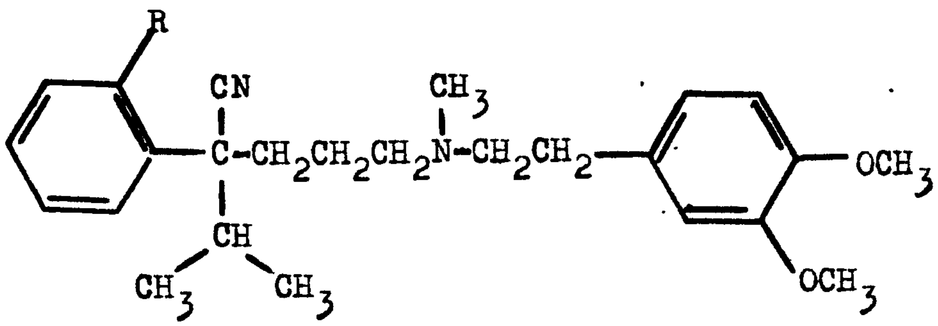

(I)

in which R is a methyl, chlorine, bromine, $C_1$ to $C_4$ alkoxyl or methylthio group, and their addition salts with pharmaceutically acceptable inorganic or organic acids.

2. 2-Isopropyl-2-(2-methylphenyl)-5-(N-methylhomoveratryl-amino)valeronitrile and its hydrochloride.

3. 2-(2-Chlorophenyl)-2-isopropyl-5-(N-methylhomoveratryl-amino)valeronitrile and its hydrochloride.

4. 2(-2-Bromophenyl)-2-isopropyl-5-(N-methylhomoveratryl-amino)valeronitrile and its hydrochloride.

5. 2-Isopropyl-2-(2-methylthiophenyl)-5-(N-methylhomoveratrylamino)valeronitrile.

6. 2-(2-Methoxyphenyl)-5-(N-methylhomoveratrylamino)-valeronitrile and its hydrochloride.

7. 2-(2-Butoxyphenyl)-2-isopropyl-5-(N-methylhomoveratryl-amino)valeronitrile and its hydrogenfumarate.

8. A process for the preparation of 2-isopropyl-2-phenyl-5-(N-methyl-2-phenylethylamino)valeronitrile derivatives of the general formula I

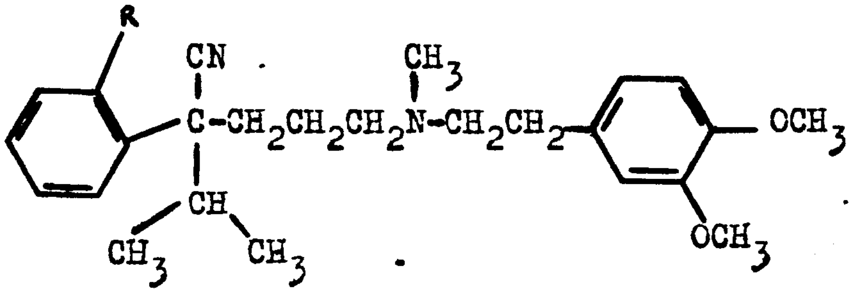

(I)

in which R is a methyl, chlorine, bromine, $C_1$ to $C_4$ alkoxyl or methylthio group, and their acid addition salts, comprising alkylation of a butyronitrile derivative of the general formula II

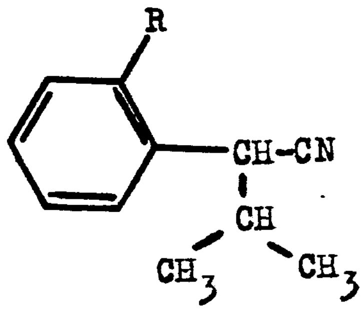

(II)

in which R has the above defined meaning, with 3,3-diethoxypropylchloride in an inert organic solvent in the presence of an alkaline condensing agent capable of binding the formed hydrogen chloride, subsequent hydrolysis of the so obtained acetal of the general formula III

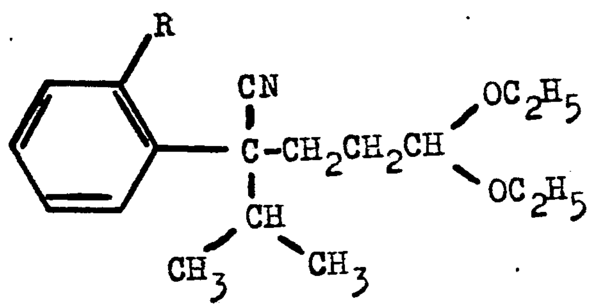

(III)

in which R is the same as above, under catalysis by an organic or inorganic acid to give an aldehyde of the general formula IV

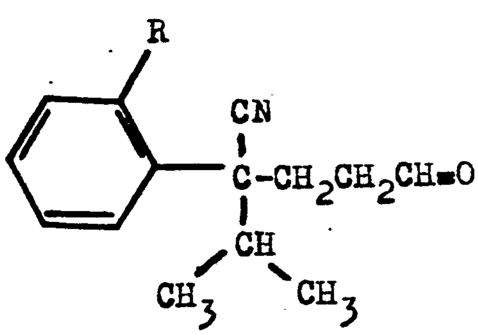

(IV)

reductive alkylation of N-methylhomoveratrylamine with the resulting aldehyde and if required neutralization of the resulting base of the general formula I with a pharmaceutically acceptable organic or inorganic acid.

9. A pharmaceutical composition comprising a valeronitrile derivative of the formula I defined in claim 1 as active component together with a pharmaceutically acceptable carrier, vehicle or adjuvant in a dosage form suitable for oral, parenteral, spray or transdermal administration.

10. A valeronitrile derivative of the formula I defined in claim 1 for use in the treatment of heart disorders.

Claims Austria

1. A process for the preparation of 2-isopropyl-2-phenyl-5-(N-methyl-2-phenylethylamino)valeronitrile derivatives of the general formula I

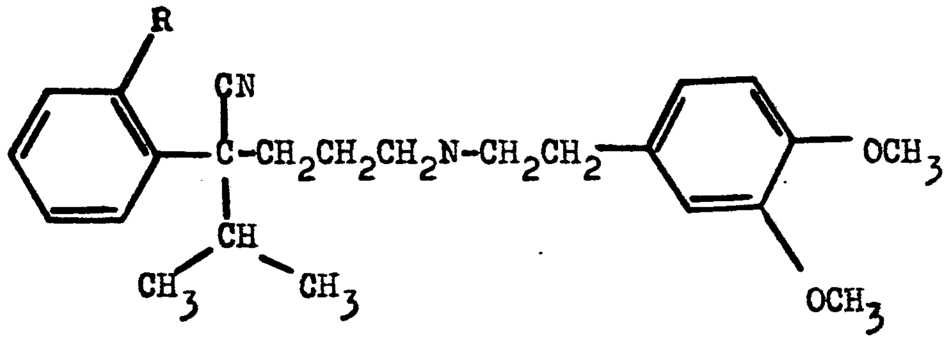

(I).

in which R is a methyl, chlorine, bromine, $C_1$ to $C_4$ alkoxyl or methylthio group, and their acid addition salts, comprising alkylation of a butyronitrile derivative of the general formula II

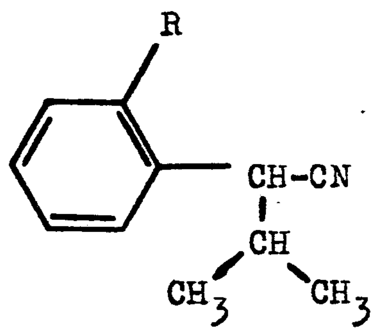

(II)

in which R has the above defined meaning, with 3,3-diethoxypropylchloride in an inert organic solvent in the presence of an alkaline condensing agent capable of binding the formed hydrogen chloride, subsequent hydrolysis of the so obtained acetal of the general formula III

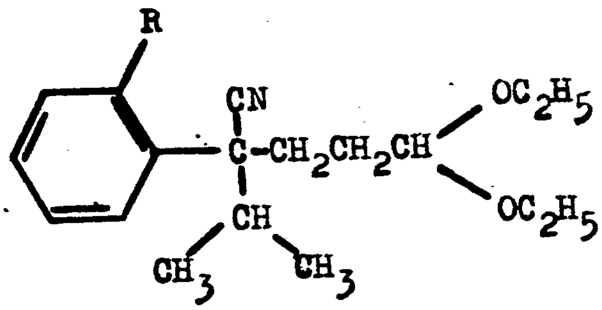

(III)

in which R is the same as above, under catalysis by an organic or inorganic acid to give an aldehyde of the general formula IV

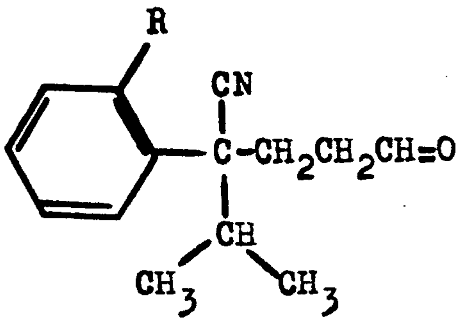

(IV)

reductive alkylation of N-methylhomoveratrylamine with the resulting aldehyde and if required neutralization of the resulting base of the general formula I with pharmaceutically acceptable organic or inorganic acid.

2. A process according to claim 1 wherein said alkylation of butyronitrile derivatives of formula II with 3,3-diethoxypropylchloride is effected in toluene in the presence of sodium amide at the boiling temperature of the reaction mixture for a period of 4 to 10 hours.

3. A process according to claim 1 wherein said hydrolysis of acetals of formula III is effected with oxalic acid dihydrate in aqueous acetone at a temperature of 20 to 40°C for 2 to 10 hours.

4. A process according to claim 1 wherein said reductive alkylation of N-methylhomoveratrylamine with aldehydes of formula IV is effected by catalytic hydrogenation over a platinum or palladium catalyst in a $C_1$ to $C_4$ aliphatic alcohol at a temperature within the range of from 20°C to the boiling temperature of the reaction mixture.

5. A process of claim 1 wherein said reductive alkylation is effected under the action of formic acid as a reducing agent in an inert organic solvent at a temperature ranging from 20°C to the boiling temperature of the reaction mixture.

6. A process of claim 5 wherein the reductive alkylation is effected with substantially 90% formic acid in toluene at the boiling temperature for a period of 1 to 2 hours.

7. 2-Isopropyl-2-phenyl-5-(N-methyl-2-phenylethylamino)-valeronitrile derivatives whenever prepared by a process substantially in accordance with any of the preceding claims or an obvious equivalent thereof.

0257768

European Patent Office

# EUROPEAN SEARCH REPORT

Application number

EP 87 30 6319

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 180 810 (BASF)<br>* Claims; example 24 *<br>--- | 1-10 | C 07 C 121/66<br>C 07 C 121/75<br>C 07 C 149/42<br>A 61 K 31/275 |
| A | EP-A-0 064 158 (BASF)<br>* Claims; page 20, example 2 *<br>--- | 1-10 | |
| A | FR-M- 2 663 (KNOLL)<br>* Abstract; page 7 *<br>----- | 1-10 | |
| | | | TECHNICAL FIELDS SEARCHED (Int. Cl.4) |
| | | | C 07 C 121/00<br>C 07 C 149/00 |

The present search report has been drawn up for all claims

| Place of search | Date of completion of the search | Examiner |
|---|---|---|
| THE HAGUE | 13-10-1987 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document